(19) **Europäisches Patentamt**

**European Patent Office**

**Office européen des brevets**

(11) Veröffentlichungsnummer: **0 120 137 B1**

(12) **EUROPÄISCHE PATENTSCHRIFT**

(45) Veröffentlichungstag der Patentschrift:
13.05.87

(51) Int. Cl.⁴: **A 61 B 5/02,** G 01 L 9/00

(21) Anmeldenummer: **83113018.2**

(22) Anmeldetag: **23.12.83**

(54) **Sensor zur Bestimmung des systolischen Blutdruckes an Versuchstieren.**

(30) Priorität: **24.02.83 DE 8305182 U**

(43) Veröffentlichungstag der Anmeldung:
**03.10.84 Patentblatt 84/40**

(45) Bekanntmachung des Hinweises auf die Patenterteilung:
**13.05.87 Patentblatt 87/20**

(84) Benannte Vertragsstaaten:
**AT BE CH DE FR GB IT LI NL SE**

(56) Entgegenhaltungen:
**DE - A - 2 848 198**
**GB - A - 2 073 889**
**US - A - 3 149 492**
**US - A - 4 122 843**
**US - A - 4 256 094**

**JOURNAL OF APPLIED PHYSIOLOGY, Band 25, Nr. 6, Dezember 1968, Seiten 772-773, US. D. A. DOWD u.a.: "A method for recording baby rat systolic blood pressures"**

(73) Patentinhaber: **Grünenthal GmbH, Zieglerstrasse 6, D-5100 Aachen (DE)**

(72) Erfinder: **Hess, Volker, Dr.-Ing., Giselastrasse 5, D-5100 Aachen (DE)**

## Beschreibung

Die experimentelle Messung des Blutdruckes an Versuchstieren ist einer der wichtigsten Schritte bei der Untersuchung neuer biologisch wirksamer Stoffe bzw. Stoffgemische auf Wirkung oder Nebenwirkungen sowie z.B. bei der Standardisierung von Zubereitungen natürlich (insbesondere pflanzlich) vorkommender Wirkstoffe. Zur Ermittlung verlässlicher Werte ist es erforderlich, dass die Messung am wachen, nicht anderweitig therapierten Tier unter Bedingungen durchgeführt wird, die beim Versuchstier keine oder allenfalls zu vernachlässigende Stressreaktionen und daraus resultierende Änderungen des Blutdruckes auslösen und die auch eine Verfolgung von wirkstoffbedingten Blutdruckänderungen über längere Zeit erlauben. Diese und weitere experimentelle Forderungen an die zur Messung einzusetzenden Geräte konnten bisher noch nicht zufriedenstellend und preisgünstig erfüllt werden.

Die Erfindung betrifft nunmehr einen, im unabhängigen Anspruch 1 definierten, neuen Sensor zur unblutigen Bestimmung des systolischen Blutdruckes an Versuchstieren, insbesondere zur Erfassung des Blutdruckpulses in den Schwanzarterien des Versuchstieres, vorzugsweise denen der Ratte. Dieser Sensor hat ein geringes Gewicht und kleine Ausmasse und wird daher nach kurzer Eingewöhnung von den Versuchstieren nicht mehr als störend, d.h. nicht als stressauslösend, wahrgenommen. Dank der geringen Masse führen darüberhinaus auch Schwanzbewegungen des Tieres nur zu sehr geringen Störsignalen, so dass dieser Sensor zur Erfassung der Blutdrucksignale auch über eine längere Versuchsdauer geeignet ist. Schliesslich ist der erfindungsgemässe Sensor einfach und preisgünstig anzufertigen, wodurch es ermöglicht wird, mit vertretbarem Kostenaufwand gleichzeitig den Effekt der zu untersuchenden Substanz an einem Tierkollektiv von z.B. 10 Tieren zu messen und so individuell bedingte Fehler der Messung auszuschalten.

Die Erfindung betrifft auch die Herstellung eines Sensors, wobei ein besonders zweckmässiges Herstellungsverfahren im unabhängigen Anspruch 14 definiert wird.

Die Erfindung und weitere damit erzielte Vorteile sollen nun anhand der Zeichnungen erläutert werden:

Fig. 1 zeigt eine Darstellung des erfindungsgemässen Sensors in Ruhestellung;

Fig. 2A und 2B stellen Längsschnitte durch zwei etwas unterschiedliche Ausgestaltungsformen des Sensors dar;

Fig. 3 gibt schematisch die Anwendung des Sensors wieder;

Fig. 4A stellt einen Querschnitt durch den Sensor in Ruhestellung dar;

Fig. 4B zeigt den Sensor im Querschnitt in einer Stellung, bei der das Messsignal ausgelöst wird;

Fig. 5A und 5B zeigen den Sensor im Querschnitt mit eingesetzten, unterschiedlichen Anpassungsstücken in Ruhestellung;

Fig. 6 stellt den Längsschnitt durch eine bevorzugte Form des Anpassungsstückes dar.

Diese Zeichnungen dienen als Beispiele zur Erläuterung des Erfindungsgegenstandes, der dadurch nicht auf die dargestellten Ausführungsformen beschränkt werden soll.

In den Figuren ist (1) ein längsgeschlitztes Rohr aus einem elastischen bzw. biegsamen und federnden Material, welches vorzugsweise dem Längsschlitz (2) gegenüberliegend eine definierte Materialschwächung (3) aufweist, die beispielsweise durch Anschleifen des Rohres in Form einer Fläche vorliegen kann. Auf dieser Fläche (3) ist ein zweckmässig in elastisches Material eingebettetes, auf Biegung reagierendes elektromechanisches Wandlerelement (4) befestigt. Die ganze Anordnung wird gegebenenfalls von einer Abdeckung (5) umschlossen, die beispielsweise aus einer an der Aussenseite elektrisch leitfähigen Folie zur elektromagnetischen Abschirmung bestehen kann. Die Abdeckung (5) kann aber auch z.B. eine elastische, dünne Folie sein, die auch als Schlauchabschnitt über die Anordnung gezogen werden und dann (in den Figuren nicht dargestellt) den Schlitz (2) mit überdecken kann. Aus dem Wandlerelement (4) wird das elektrische Signal mittels eines abgeschirmten Kabels (6, 7) zu einer – nicht zum Gegenstand der Erfindung gehörenden – elektrischen Zwischenschaltung und von da aus zur eigentlichen Beobachtungs-, Messoder Registrierstation geleitet, wobei die Abschirmung des Kabels (6) über die Verbindung (7) mit der gegebenenfalls vorhandenen leitfähigen Folie (5) oder auch, wenn das Rohr (1) aus Metall besteht, mit diesem elektrisch leitend verbunden ist.

Das Wandlerelement (4) ist vorzugsweise ein auf Biegung reagierender Streifen aus piezoelektrischem keramischem Werkstoff, jedoch kommen auch andere auf Biegung ansprechende Wandlerprinzipien, wie beispielsweise kapazitive, resistive, induktive oder optoelektrische in Betracht.

Das bevorugte piezoelektrische Wandlerelement (4) wird z.B. auf die Fläche (3) geklebt oder gelötet, wobei, wenn das Rohr (1) aus einem elastischen, nicht lösbaren Metall besteht, vorzugsweise ein elektrisch leitender Kleber verwendet wird.

Wie in Figur (2A), also einem Längsschnitt in der Schnittebene A–B, dargestellt, können auch zwei parallel geschaltete Wandlerelemente (4) und 4', die über (8) miteinander leitend verbunden sind, auf dem Rohr (1) befestigt werden. Diese können dann mit einem elastischen Material (9) vergossen werden.

Besonders zweckmässig ist es, entsprechend der in Figur (2B) dargestellten Ausbildungsform, das Rohr (1) z.B. aus einem elastischen Kunststoff zu fertigen und dann direkt eine Verdickung (10), die Ausnehmungen (11) und 11' zum Einbau der Wandlerelemente (4) und (4') aufweist, anzuformen, wobei diese Verdickung einschliesslich der Ausnehmungen dann elektrisch leitend ausgerüstet werden kann. In diesem Fall wird gegebenenfalls im Rohrinneren unterhalb der Verdickung (10) eine Kerbe angebracht, um die erforderliche Materialschwächung zu bewirken, man kann aber auch direkt das Kunststoffrohr (1) so herstellen, dass dieses unterhalb des Sitzes der Wandlerelemente (4) und (4') dünnwandiger als an den Seitenteilen ist.

Ein besonders zweckmässiges Herstellungsverfahren eines erfindungsgemässen Sensors besteht darin, dass man die mit den erforderlichen elektrisch leitenden Verbindungen (6, 7 und 8) versehenen Wandlerelemente (4) und (4') an die Innenwand einer mit Kern versehenen Hohlform bringt und dann das – zunächst gegebenenfalls noch nicht den Längsschlitz (2) aufweisende – Rohr (1) mit der angeformten Verdickung (10) aus Kunststoff giesst und gleichzeitig dabei die Wandlerelemente (4) und (4') darin einbettet. Nach Entnahme aus der Form wird dann gegebenenfalls noch der Längsschlitz (2) eingesägt bzw. eingefräst. Wird bei diesem Vorgehen durch entsprechende Gestaltung der Form dafür Sorge getragen, das das Rohr exzentrisch, also unterhalb des Sitzes der Wandlerelemente (4) und (4') dünnwandiger als an den Seitenteilen ist, so ist die Herstellung des erfindungsgemässen Sensors besonders wenig arbeitsaufwendig, da – abgesehen von der Erstellung der elektrisch leitenden Verbindungen (6, 7 und 8) – keinerlei Montagearbeiten oder dergleichen erforderlich werden.

Weitere im Rahmen der Erfindung liegende Ausbildungsformen des erfindungsgemässen Sensors ergeben sich für den Durchschnittsfachmann von selbst, d.h. ohne erfinderische Tätigkeit aus den hier gemachten Angaben und/oder den anliegenden Zeichnungen. So kann beispielsweise die (praktisch keinen Widerstand auslösende) Luft in dem Spalt (2) durch eine hochelastische, d.h. eine der Biegung des Sensors möglichst wenig Widerstand entgegensetzende Masse ausgefüllt sein, ohne dass damit eine Änderung des Konstruktionsprinzips des erfindungsgemässen Sensors erfolgt.

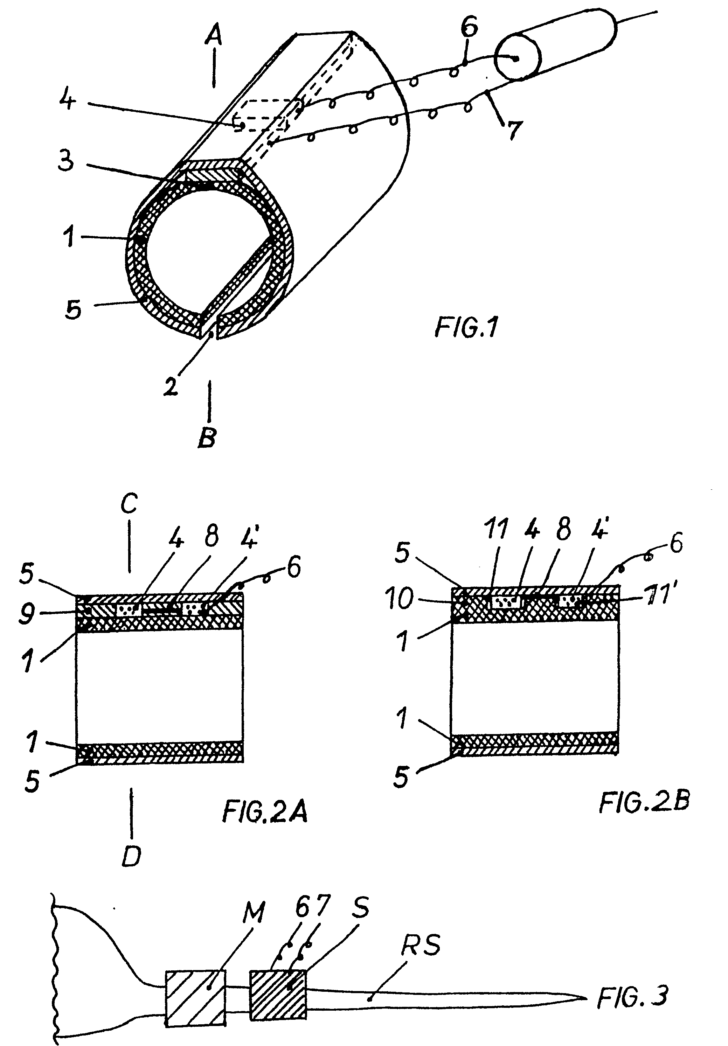

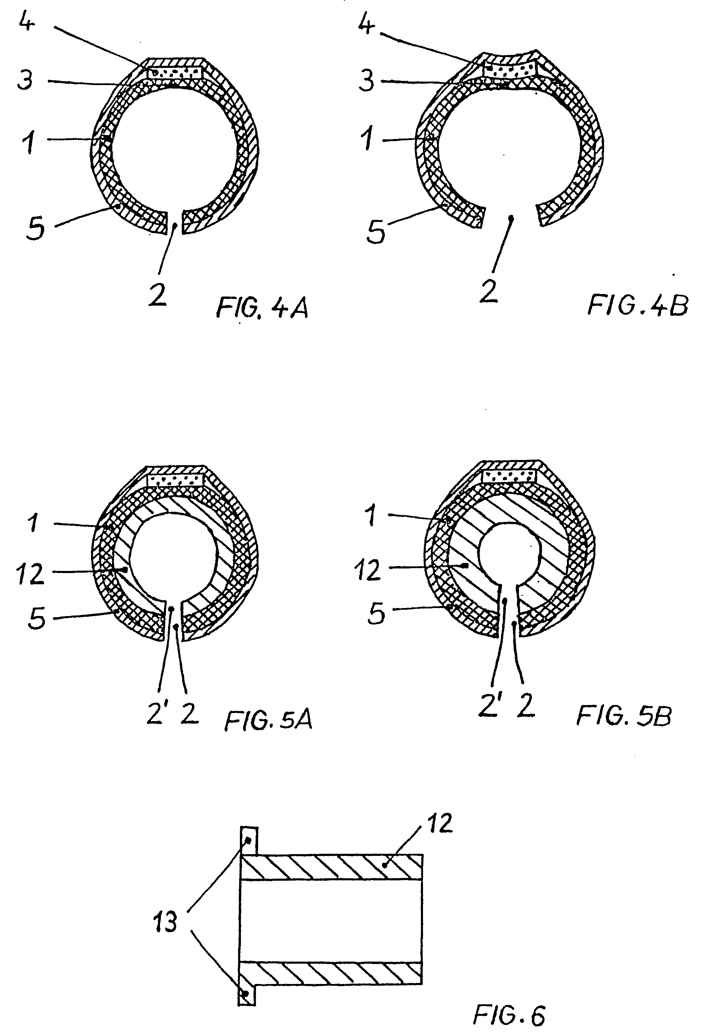

Zur Blutdruckmessung beispielsweise an der Ratte werden die Arterien des Rattenschwanzes (RS in Fig. 3) mittels einer Druckmanschette (M) an der Schwanzbasis suprasystolisch komprimiert. Der hinter der Manschette (M) auf den Rattenschwanz aufgeschobene Sensor (S) registriert dann keine Pulsationen mehr. Bei langsamem Absenken des Druckes in der Manschette werden kurz nach Unterschreiten des systolischen Druckes wieder Pulsationen, die Querschnittsänderungen des Schwanzes und damit eine im Bereich der Materialschwächung (3) eng lokalisierte Biegung bedingen (wie dies in Fig. 4A und 4B, also Querschnitten in der Schnittebene C–D, angedeutet ist), angezeigt.

Entsprechend der Grösse des Versuchstieres variiert auch der Schwanzdurchmesser und insbesondere bei chronischen Versuchen, z.B. über mehrere Wochen müssen daher aufgrund des Wachstums der Tiere bekannte Pulssensoren gegen solche mit grösserer lichter Weite ausgetauscht werden. Dies bedingt, dass man je Versuchstier über einen ganzen Satz von Sensoren verfügen musste, die beispielsweise mit Innendurchmessern von 5 bis 10 mm mit einer Abstufung von je 0,5 mm Innendurchmesser (also mit 11 Sensoren je Satz) angeboten werden. Der erfindungsgemässe Sensor kann demgegenüber jeder in Betracht kommenden Schwanzdicke angepasst werden, indem man, wie in Fig. 5A und 5B dargestellt, in das Rohr (1) Anpassungsstücke (12) einsetzt, die einen dem Schwanzdurchmesser des jeweiligen Versuchstieres entsprechenden Innendurchmesser haben und ihrerseits, wie das Rohr (1), einen Längsschlitz (2') aufweisen. Diese Anpassungsstücke übertragen die Blutdruckpulsation auf das Rohr (1), so dass dann auf die Biegung im Bereich der Materialschwächung (3) das Wandlerelement (4) anspricht.

Vorzugsweise sind an einer Seite des Anpassungsstückes (12) einige Nasen (13), wie in Fig. 6 dargestellt, angeformt, die bei der Applikation des Sensors in Richtung der Manschette (M) zu liegen kommen. Diese Nasen (13) erleichtern das passgenaue Aufschieben des Sensors mit eingesetztem Anpassungsstück und verhindern, dass, zumal bei längeren Versuchen, das Anpassungsstück (12) aus dem Rohr (1) herausrutscht oder z.B. von dem Versuchstier herausgezogen werden kann.

Der erfindungsgemässe Sensor kann in nahezu beliebiger Grösse (Aussendurchmesser und Länge) gefertigt werden, wobei aber praktische Gesichtspunkte und insbesondere die Forderung, dass die Tiere durch den Sensor möglichst wenig gestört werden sollen, gewisse Grenzen für die tatsächlich zu wählenden Masse setzen. Vorzugsweise ist die Länge des erfindungsgemässen Sensors wenigstens gleich dessen Aussendurchmesser. Seine bevorzugte Höchstlänge entspricht etwa dem Biegeradius des Schwanzes an der Messstelle, da dann der Schwanz des Versuchstieres in seiner Beweglichkeit praktisch nicht beeinträchtigt wird und somit bei dem Tier keine Abwehr- bzw. Befreiungsaktionen und daraus resultierende Stressreaktionen ausgelöst werden. Diese bevorzugte Höchstlänge ist darüberhinaus messtechnisch von Bedeutung, da dann durch ein Verbiegen des Schwanzes keine Kräfte auf den Sensor einwirken, die die blutdruckbedingten Änderungen des Durchmessers und damit die Anzeige überlagern oder verfälschen könnten.

**Patentansprüche**

1. Sensor zur Bestimmung des systolischen Blutdruckes an Versuchstieren, dadurch gekennzeichnet, dass an der Aussenseite eines längsgeschlitzten Rohres (1) aus elastischem Material an einer vorzugsweise dem Längsschlitz (2) gegenüberliegenden Stelle definierter Materialschwächung (3) ein auf Biegung reagierendes elektromechanisches Wandlerelement (4) befestigt ist und gegebenenfalls diese Anordnung von einer Abdeckung (5) umschlossen ist.

2. Sensor gemäss Anspruch 1, dadurch gekennzeichnet, dass die Ableitung für das Signal aus dem Wandlerelement (4) aus einem Koaxialkabel (6, 7) besteht.

3. Sensor gemäss einem der Ansprüche 1 oder 2, dadurch gekennzeichnet, dass das Wandlerelement (4) ein auf Biegung reagierender Streifen aus piezoelektrischem keramischem Material ist.

4. Sensor gemäss einem der Ansprüche 1 oder 2, dadurch gekennzeichnet, dass das Wandlerelement (4) aus zwei oder mehreren parallel geschalteten, auf Biegung reagierenden Streifen aus piezoelektrischem keramischem Material aufgebaut ist.

5. Sensor gemäss einem der Ansprüche 1 bis 4, dadurch gekennzeichnet, dass die Stelle definierter Materialschwächung (3) eine vorzugsweise durch Anschleifen des Rohres (1) gebildete Fläche darstellt, auf der das Wandlerelement (4) befestigt ist.

6. Sensor gemäss einem der Ansprüche 1 bis 5, dadurch gekennzeichnet, dass das Wandlerelement (4) in elastisches Material (9) eingebettet ist.

7. Sensor gemäss einem der Ansprüche 1 bis 6, dadurch gekennzeichnet, dass als äussere Abdeckung (5) eine an der Aussenseite elektrisch leitfähige Folie verwendet wird.

8. Sensor gemäss einem der Ansprüche 1 bis 7, dadurch gekennzeichnet, dass zur Ableitung des vom Wandlerelement (4) erzeugten elektrischen Signals ein abgeschirmtes Kabel vorgesehen ist.

9. Sensor gemäss einem der Ansprüche 1 bis 8, dadurch gekennzeichnet, dass das Rohr (1) aus Kunststoff besteht und eine angeformte Verdickung (10) aufweist, in der Ausnehmungen (11) zur Aufnahme des Wandlerelementes (4) vorgesehen sind.

10. Sensor gemäss einem der Ansprüche 1 bis 9, dadurch gekennzeichnet, dass seine Länge wenigstens gleich seinem Aussendurchmesser ist.

11. Sensor gemäss einem der Ansprüche 1 bis 10, dadurch gekennzeichnet, dass er kürzer als oder gleich lang wie der Biegeradius des zur Messung des systolischen Blutdruckes herangezogenen Schwanzes des Versuchstieres ist.

12. Sensor gemäss einem der Ansprüche 1 bis 11, dadurch gekennzeichnet, dass sein Innendurchmesser durch in das Rohr (1) eingesetzte längsgeschlitzte Anpassungsstücke (12) variiert wird.

13. Sensor gemäss Anspruch 12, dadurch gekennzeichnet, dass an das Anpassungsstück (12) Nasen (13) angeformt sind.

14. Verfahren zur Herstellung eines Sensors gemäss Anspruch 9, zurückbezogen auf Anspruch 6, dadurch gekennzeichnet, dass das Rohr (1) mit der angeformten Verdickung (10) unter gleichzeitiger Einbettung eines mit elektrisch leitenden Verbindungen (6, 7 und 8) ausgerüsteten ein- oder mehrteiligen Wandlerelementes (4, 4') aus Kunststoff gegossen wird und gegebenenfalls erst anschliessend der Längsschlitz (2) eingesägt oder eingefräst wird.

**Claims**

1. Sensor for measuring the systolic blood pressure of laboratory animals, characterized in that a flexure-sensitive electromechanical transducer element (4) is attached to the outside of a resilient tube (1) which has been slit longitudinally, attachement being effected at a position (3) where the strength of the tube material has been reduced by a defined amount, this position (3) preferably being opposite the longitudinal slit (2), and the complete assembly being enclosed by a covering (5) if the particular circumstances so require.

2. Sensor according to claim 1, characterized in that the output line for the signal from the transducer element (4) consists of a coaxial cable (6, 7).

3. Sensor according to claim 1 or 2, characterized in that the transducer element (4) is a flexure-sensitive strip which is composed of a ceramic material exhibiting piezoelectric properties.

4. Sensor according to claim 1 or 2, characterized in that the transducer element (4) is constructed by parallel-connecting two or more flexure-sensitive strips which are composed of a ceramic material exhibiting piezoelectric properties.

5. Sensor according to any one of claims 1 to 4, characterized in that a flat area distinguishes the position (3) where the strength of the tube material has been reduced by a defined amount, this flat area preferably being formed by grinding the tube (1) and serving as the surface to which the transducer element (4) is attached.

6. Sensor according to any one of claims 1 to 5, characterized in that the transducer element (4) is embedded into a resilient material (9).

7. Sensor according to any one of claims 1 to 6, characterized in that a film possessing an electrically conductive outer surface is used as the external covering (5).

8. Sensor according to any one of claims 1 to 7, characterized in that a screened cable is provided for leading off the electrical signal which is generated by the transducer element (4).

9. Sensor according to any one of claims 1 to 8, characterized in that the tube (1) is composed of a plastic, and possesses a moulded-on, thickened portion (10), in which recesses (11) for receiving the transducer element (4) are provided.

10. Sensor according to any one of claims 1 to 9, characterized in that its length is at least equal to its outside diameter.

11. Sensor according to any one of claims 1 to 10, characterized in that its length does not exceed the bending radius of the tail which is being used for measuring the systolic blood pressure of the laboratory animal to which it belongs.

12. Sensor according to any one of claims 1 to 11, characterized in that its inside diameter is varied by means of longitudinally-slit fitting pieces (12), which are inserted into the tube (1).

13. Sensor according to claim 12, characterized in that lugs (13) are moulded onto the fitting piece (12).

14. Process for manufacturing a sensor according to claim 9, referred-back to claim 6, characterized in that the tube (1), with the moulded-on, thickened portion (10), is cast in a plastic, while at the same time embedding a transducer element (4, 4') which may consist of one piece, or may comprise several pieces, and which is equipped with electrically conducting connections (6, 7 and 8), while the longitudinal slit (2) may not be sawn or milled until after the casting operation has been completed, according to the particular circumstances.

**Revendications**

1. Capteur de tension artérielle systolique pour animaux de laboratoire, caractérisé par un élément transducteur (4) électromécanique réagissant à la flexion, fixé sur la paroi extérieure d'un tuyau (1) en matière élastique pourvu d'une fente longitudinale (2), dans une zone de plus faible épaisseur (3) prédéterminée, située de préférence du côté opposé à la fente longitudinale, ce dispositif étant fermé en tant que de besoin par un recouvrement (5).

2. Capteur selon la revendication 1, caractérisé en ce que le conducteur du signal émis par l'élément transducteur (4) est constitué par un câble coaxial (6, 7).

3. Capteur selon l'une des revendications 1 et 2, caractérisé en ce que l'élément transducteur (4) est constitué d'une bande en matière céramique piézoélectrique réagissant à la flexion.

4. Capteur selon l'une des revendications 1 et 2, caractérisé en ce que l'élément transducteur (4) est constitué de deux ou plusieurs bandes, connectées en parallèle, en matière céramique piézoélectrique réagissant à la flexion.

5. Capteur selon l'une des revendications 1 à 4, caractérisé en ce que la zone de plus faible épaisseur (3) prédéterminée est formée par une surface plane, obtenue de préférence par un polissage du tuyau (1), sur laquelle est fixé l'élément transducteur (4).

6. Capteur selon l'une des revendications 1 à 5, caractérisé en ce que l'élément transducteur (4) est inséré dans du matériau élastique (9).

7. Capteur selon l'une des revendications 1 à 6, caractérisé en ce que le recouvrement extérieur (5) est constitué par une feuille à surface extérieure conductrice.

8. Capteur selon l'une des revendications 1 à 7, caractérisé en ce que le signal électrique émis par l'élément transducteur (4) est conduit par un câble protégé.

9. Capteur selon l'une des revendications 1 à 8, caractérisé en ce que le tuyau (1) en matière synthétique comporte une surépaisseur (10) formée dans la matière, dans laquelle sont prévus des évidements (11) recevant l'élément transducteur (4).

10. Capteur selon l'une des revendications 1 à 9, caractérisé en ce que sa longueur est au moins égale à son diamètre extérieur.

11. Capteur selon l'une des revendications 1 à 10, caractérisé en ce que sa longueur est plus petit ou égale au rayon de courbure de la queue de l'animal cobaye, utilisée pour déterminer la tension artérielle systolique.

12. Capteur selon l'une des revendications 1 à 11, caractérisé en ce que son diamètre peut varier grâce à des éléments adaptateurs (12) pourvus d'une fente longitudinale, insérés dans le tuyau (1).

13. Capteur selon la revendication 12, caractérisé en ce que l'élément adaptateur (12) comporte des saillies (13) formées dans la matière.

14. Procédé de fabrication d'un capteur conforme à la fig. 9, avec référence à la revendication 6, caractérisé en ce que le tuyau (1) et sa surépaisseur (10) formée dans la matière sont coulés en matière synthétique avec insertion simultanée d'un élément transducteur (4, 4') en une ou plusieurs parties, pourvu de raccords électriques conducteurs (6, 7, 8), la fente longitudinale (2) étant sciée ou fraisée en tant que de besoin ultérieurement.

0120137

FIG.1

FIG.2A

FIG.2B

FIG.3

FIG. 4A

FIG. 4B

FIG. 5A

FIG. 5B

FIG. 6